# EUROPEAN PATENT SPECIFICATION

(11) **EP 0 576 285 B1**
(45) Date of publication and mention of the grant of the patent: **03.12.1997**
(21) Application number: 93304956.1
(22) Date of filing: 24.06.1993
(51) Int. Cl.: B41J 2/205

(54) **Ink jet recording method and apparatus**
Tintenstrahlaufzeichnungsverfahren und -gerät
Méthode et appareil pour l'enregistrement à jet d'encre

(30) Priority: 26.06.1992 JP 169522/92
(43) Date of publication of application: 29.12.1993
(73) Proprietor: CANON KABUSHIKI KAISHA, Tokyo (JP)
(72) Inventor: Ono, Takeshi, c/o Canon Kabushiki Kaisha, Tokyo (JP)
(74) Representative: Beresford, Keith Denis Lewis

(56) References cited:
- EP-A- 0 288 044
- EP-A- 0 317 140
- WO-A-92/04191
- GB-A- 2 112 980
- US-A- 4 561 025
- US-A- 4 710 784

## Description

The present invention relates to ink-jet recording methods and apparatuses used for forming images of input data such as characters, pictures, drawings or the like on a recording medium.

Recently, the ink-jet recording method has been increasingly attracting attention in the field of a data processing, and it comprises the step of forming an image of input data of characters, pictures, drawings or the like on a recording medium (e.g a sheet of paper, an OHP sheet or a sheet of cloth) by directly ejecting ink droplets from minute nozzles. Therefore, ink-jet recording apparatuses including recording means for performing such method have been used in data processing systems: they have been provided as printers as output terminals of copying machines, facsimiles, printing machines, word processors, work stations, or the like; or they have been provided as handy- or portable-printers of personal computers, host computers, optical disc- or video-equipment or the like.

The recording means (e.g., a recording head) comprises a plurality of recording elements which are arranged in a direction perpendicular to a scanning direction of the recording means. Each recording element has a liquid passage with a recording element (e.g., a thermal element). The liquid passage communicates to a minute nozzle (e.g., an ink ejection outlet) through which ink droplets can be successively ejected to a surface of the recording medium in response to a command corresponding to the input data. The recording element is provided in the liquid passage to generate thermal energy to cause sudden volume change of the ink around the recording element. Consequently an ink droplet is pushed out from the minute nozzle.

In the conventional ink-jet recording apparatus, a certain amount of input data corresponding to one page of the information to be recorded as an image is referred as image data which is divided into several blocks, i.e., the block is an aliquot part of the image data enough to form one line of the page by one scanning movement of the recording head. In this specification, the scanning movement of the recording head is defined as a movement of the recording head for printing one block of the image data so as to form a line of the page in a direction perpendicular to a transporting direction of the recording medium, In this specification, furthermore, the transporting direction of the recording medium is referred as a sub-scanning direction while the direction perpendicular to the transporting direction is referred as a main-scanning direction. Therefore the recording head prints one block of the.image data to form one line of the page on the recording medium by performing one scanning movement thereof. By repeating the scanning movements of the recording head, the data corresponding to one page of the information can be recorded on the recording medium. After preparing one block of the image data for the one scanning movement, the recording head starts the scanning movement at a constant speed. During the recording, a plurality of dots is successively putted on a surface of the recording medium by ejecting ink droplets from the recording head. Therefore the block can be defined as a unit of the image data for recording by one scanning movement of the recording head. In general, a size of the block is limited by a capacitive condition of the image memory at a time of directly before the scanning movement, or it is limited by other factors such as the amount of dots to be formed by the recording head of a semimulti-type, a size of the recording medium, a moved distance of the carriage from a starting point, or the like.

In this type of the ink-jet recording apparatus, the recording elements are driven all at once, or block by block consisting of a certain number of the recording elements by applying voltage pulses of a certain width in sequence. In general, it is important to control the pulse width so that each pulse gives just sufficient energy for ejecting ink so that excess energy is not produced. This is important not only for energy saving but also for stabilizing the ink ejection in the course of repetitive drive of the recording head.

In this type of the ink-jet recording apparatus, furthermore, there are two printing modes: a normal mode for directly recording input data just as it is; and a draft mode for recording input data after performing a thinned-out processing in which some dots are removed in accordance with a certain rule or with a certain period for the purpose of: saving the ink; shortening a printing period; or performing a high-speed printing. According to such thinned-out processing, for example, adjacent dots are not printed at the same time so as to a checkered pattern is formed. These modes can be selected by setting the bit switch at a time of switching the electric power on, or they can be selected by operating the key on the operation panel at intervals of forming images corresponding to respective pages of the recording medium.

EP0317140 discloses an ink jet recording apparatus which has a standard printing mode and another printing mode in which thinned out printing is carried out. Thinned out printing characters are pre-stored for each normal printing character and thinned out printing is carried out in response to the selection of a particular mode of printing by the user.

In spite of the above advantageous features of the conventional ink-jet recording apparatus, however, the conventional recording apparatus cannot select such modes at intervals of forming parts of an image corresponding to respective lines of the page. Therefore a large amount of ink is ejected in spite of that an image of the block having a lot of black dots should be formed, and resulting that a surface of the recording medium becomes wet with the ink and wrinkles easily. In the case of that the draft mode is selected at all times, on the other hand, a small amount of ink is ejected in spite of that an image of the block having a few black dots should be formed, and resulting that an obtained image is hard to read because the characters are blurred.

Furthermore, the conventional ink-jet recording apparatus does not have any means for controlling potencies of the energies used for ejecting ink droplets during a process of recording one block of the image data. Consequently, the recording head can be gradually over heated and a viscosity of the ink to be ejected from the orifices can be gradually decreased when a percentage of the black dots in one block or the prescribed amount of the image data for the one scanning movement of the recording head is comparatively at a higher level (over 50%) during the one block recording. Therefore a volume of the ink droplet tends to rise during the printing and the quality of recorded images can be deteriorated.

In a first aspect of the present invention, there is provided an ink-jet recording apparatus as set out in claim 1 and a method for recording an image on a recording medium as set out in claim 11.

A recording method and apparatus embodying the present invention can achieve high quality recorded images by making it possible to perform optimum control of the (recording) element drive.

The above and other effects, features and advantages of the present invention will become more apparent from the following description of embodiments thereof taken in conjunction with the accompanying drawings.

Embodiments of the invention will now be described, by way of example, and with reference to the accompanying drawings in which:
Fig. 1 is a schematic perspective view showing an example of an ink-jet cartridge used by an ink-jet recording apparatus in accordance with the present invention;
Fig. 2 is a schematic perspective view showing an ink-jet printer used by an ink-jet recording apparatus in accordance with the present invention;
Fig. 3 is a block diagram showing the details of the ink-jet recording apparatus in accordance with the first preferred embodiment of the present invention;
Fig. 4 is a graphical representation of the relation between the pulse width and the temperature of the recording head;
Fig. 5 is a general flow chart of a recording process controlled by the main control unit and used for forming the image of data corresponding to one page of the recording medium;
Fig. 6 is a general flow chart of a recording process controlled by the recording control unit and used for forming the image of one block of data;
Fig. 7 is a block diagram showing the details of the control portion in the ink-jet recording apparatus in accordance with the second preferred embodiment of the present invention; and
Fig. 8 is a general flow chart of the recording process controlled by the control unit of the second embodiment.

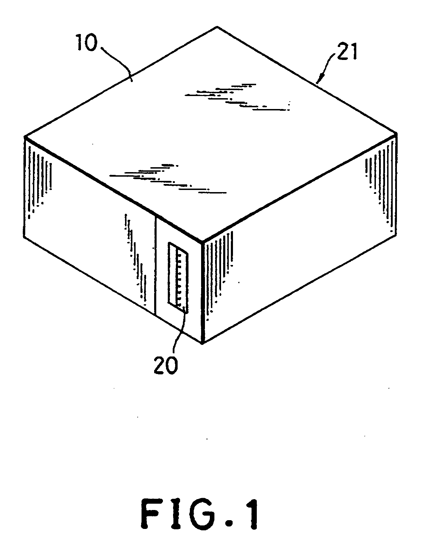

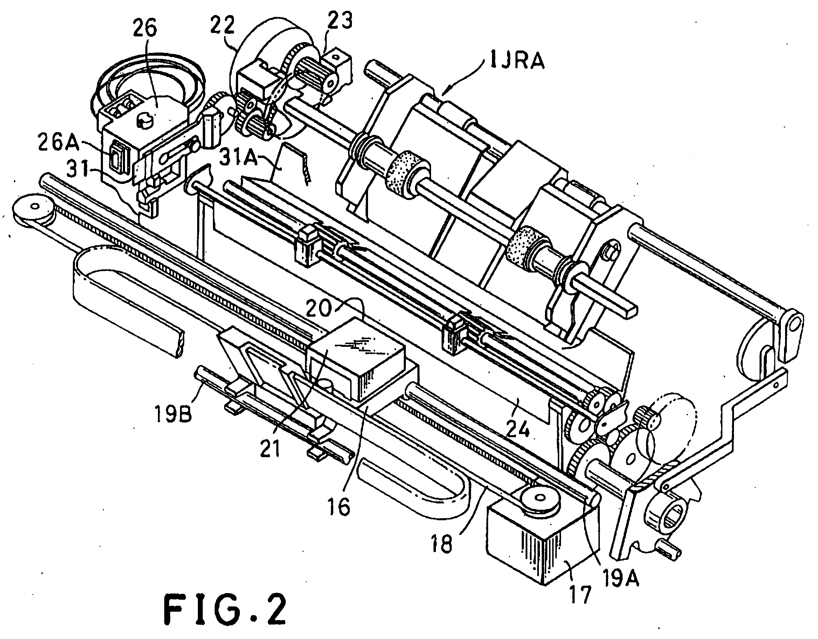

Referring now to the attached drawings for a more complete understanding of the invention, Figs. 1 and 2 show a first preferred embodiment of a novel ink-jet recording apparatus to be provided as a recording system in a facsimile in accordance with the present invention.

In these figures, reference numeral 21 designates an ink-jet cartridge. The cartridge 21 is composed of an ink-jet recording head 20 and an ink tank 10 and is formed as a disposable type one which can be removably secured to a carriage 16 of the ink-jet recording apparatus (IJRA).

The ink-jet recording head 20 comprises a plurality of recording elements which are arranged in a direction perpendicular to a scanning direction of the recording head 20. Each recording element has a minute nozzle, a liquid passage and a thermal element. The recording head 20 is in the type of ejecting ink droplets from each minute nozzle formed in the tip thereof to a surface of the recording medium such as a sheet of recording paper (i.e, a record sheet) by forming a bubble in the ink by applying a thermal energy generated by the thermal element. In this embodiment, the tip of the recording head 20 is slightly protruded from a surface of the cartridge 21.

The ink tank 10 is composed of an ink absorbent; a container for holding the absorbent with ink; and a cover for sealing an opening of the container (these elements are not shown in the figures). The ink tank 10 is filled with ink and supplies the ink little by little to the ink-jet recording head 20 so as to smoothly eject ink droplets from each nozzle of the head 20.

The ink jet cartridge 21 thus constructed is removably mounted on a carriage of the ink jet recording apparatus IJRA to be described later in a predetermined manner so that a desired image is recorded on a record sheet by relative movement between the carriage HC and the record sheet in accordance with an input image data.

Reference numeral 20 denotes an ink jet head (record head) of the ink jet head cartridge 21 having nozzles which eject ink to a record plane of the record sheet fed onto a platen 24 by feed means which is driven by a paper feed motor to be described later. Reference numeral 16 denotes a carriage for holding the ink jet cartridge 21.

The carriage 16 is connected with a part of a driving belt 18 for transmitting the drive force of a carriage motor 17 to the carriage 16 so that the carriage 16 moves along two guide shafts 19A and 19B. These shafts 19A and 19B are parallel provided each other to keep a stable movement of the carriage 16. Therefore the recording head 20 can be reciprocally moved in an extent of a total width of a record plane of the record sheet on a platen 24, along which the recording head 20 forms an image in accordance with the input data. At intervals of the scanning movements, the record sheet is transported step by step with a prescribed length in a direction perpendicular with the main-scanning direction.

Reference numeral 26 denotes a head recovering member being arranged on a position facing to one end of a path on which the recording head 20 can be moved forth and back in a direction perpendicular to the transporting direction (i.e., the sub-scanning direction) of the record sheet. This end of the path is defined as "a home position" of the recording head. The head recovering system 26 is driven by a driving force of a driving motor 22 via a transmission mechanism 23 and performs a capping operation. In the capping operation, the recording head 20 is stayed at the home position and is capped with a capping member 26A of the head recovering system 26.

In conjunction with the capping operation, a process of removing ink with a high viscosity or eliminating the ejection failure caused by drying or caking of ink in the liquid passages or near the nozzles of the recording head 20 is performed so as to smoothly eject ink droplets. These undesirable ink portions can be forcibly removed by sucking means such as a suction pump installed in the head recovering system 26. The recording head 20 can be protected by capping the head with the capping member 26A when the recording process is completed. These processes are carried out in the case of that an electric power source is switched on; the recording head is replaced by new one; or a non-recording state continues for a long time or particular orifices are not used for long time owing to the arrangement or printed images.

Adjacent to the head recovering system 26, a blade 31 is disposed in such a manner that it can project toward the region where the recording head 20 can move. The blade 31 is made of silicone rubber and is held by a blade holding means 31A. The blade 31 is used to wipe the orifice disposed face after performing the ejection recovery process or the like so that wet or paper particulate contaminates are removed therefrom. Such wiping motion is carried out by a force caused by the driving motor 22 and the driving mechanism 23 used for driving the recovering system 26.

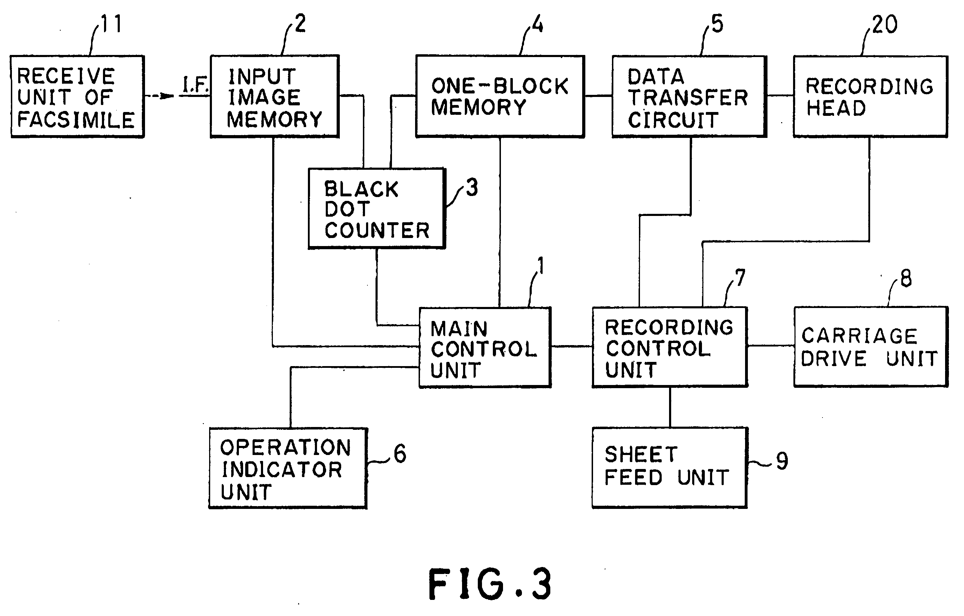

Fig. 3 is a block diagram showing an arrangement of a control system of the recording apparatus of the first preferred embodiment.

Reference numeral 1 denotes a main control unit of the recording apparatus. The main control unit 1 is responsible for controlling the recording apparatus as a whole so as to perform data processing.

Reference numeral 2 denotes an input memory for accepting image data from a host apparatus functioning as the source of the image data. In this embodiment, the host apparatus is a receiving unit of the facsimile 11 The input memory 2 transfers the image data to a one-block image memory 4 while a black-dot counter 3 counts the total number of black dots in one block of the image data. The count can be also performed by a software on the CPU in the main control unit. In general, the CPU is connected to an ROM that stores various programs executed by the CPU, and also the CPU is connected to an RAM used as working areas (CPU, ROM and RAM are not shown in the figure).

In this embodiment, the ink-jet recording apparatus comprises recording head which is in the type of a semi-multi typed head having nozzles corresponding to dozens of dots and using thermal energy for ejecting ink from a minute nozzle by causing sudden volume change of the ink around the recording element (i.e, thermal element which is responsible for generating the thermal energy). The recording head records data corresponding to one page of the information on a record sheet by several scanning movements.

Reference numeral 5 denotes a data transfer circuit which is responsible for transferring the image data from the one-block image memory 4 to the recording head 20.

Reference numeral 7 denotes a recording control unit composed of one tip micro-computer. The recording control unit 7 controls a process of transferring the data from the circuit 5 to the recording head 20 and a process of driving the head 20 in accordance with the provided data. The recording control unit 7 is also connected to a carriage drive unit 8 and a recording medium transfer unit (a sheet feed unit) 9. The recording control unit 7 sends control signals to these units 8 and 9 for driving the recording head 20 with an appropriate manner. The carriage drive unit 8 is composed of a carriage motor and a motor driver, while the recording medium transferring unit 9 is composed of a feed motor and a motor driver.

Reference numeral 6 denotes an operation indicator unit having key switches, LED, LCD and the like for indicating the condition of the recording apparatus.

Reference numeral 11 denotes a receiving unit of the facsimile. This unit 11 is able to receive image data from everywhere through the lines. The received data is restored and then it is transferred to the input image memory 2 through the interface (I.F).

In this embodiment, the recording head 20 has a thermometer for measuring the temperature of an inner side of the recording head 20 for driving the thermal elements of the head with the optimum driving pulse width.

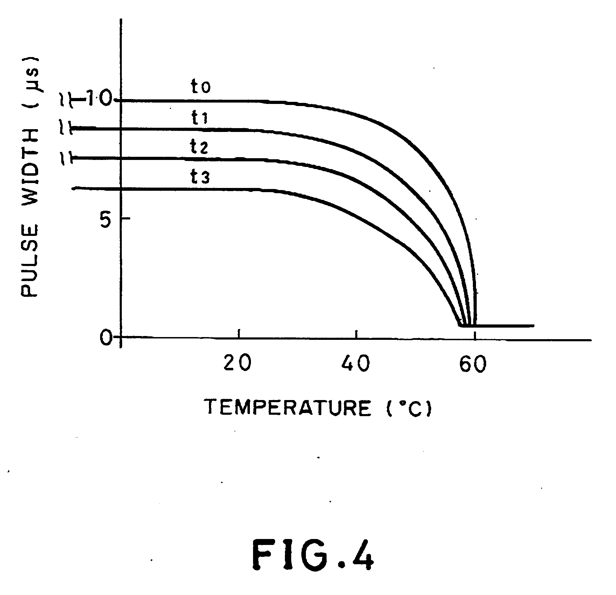

Fig. 4 indicates four different table of the voltage pulse width, which are written in the ROM. In the figure, a vertical line indicates the pulse width and a horizontal line indicates the temperature of the recording head. These different table of the pulse width (t₀-t₃) in the range of 10 to 6 µS can be selected and changed by the ROM during each scanning movement of the recording head in accordance with a different number of black dots in each block.

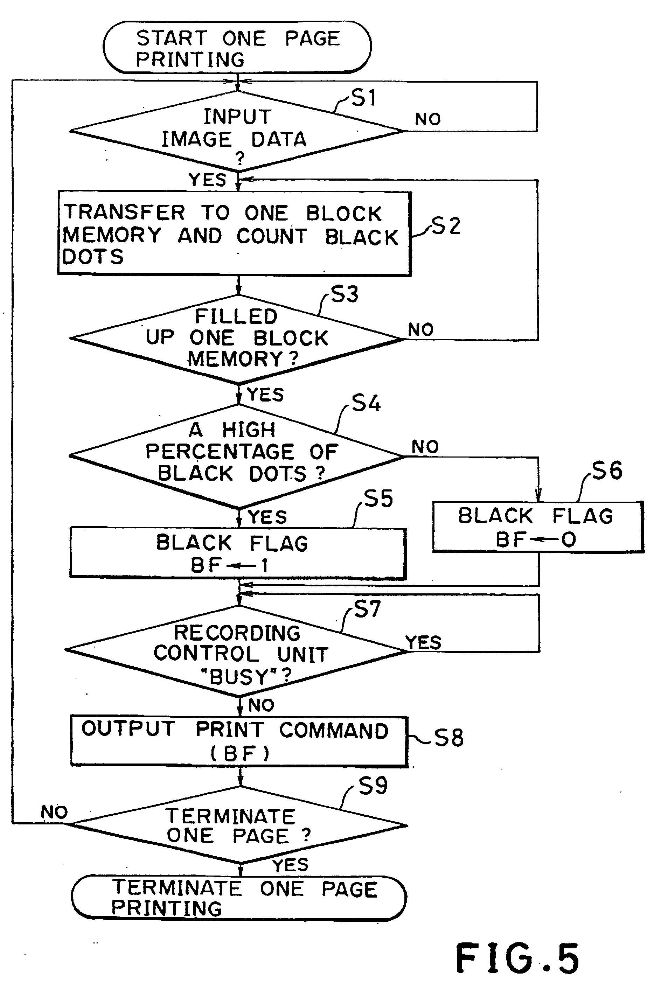

Fig.5 is a general flow chart of a recording process controlled by the main control unit 1 and used for forming an image of data corresponding to one page of the recording medium. The recording process comprises the following steps:
(1) In step 1 (S1), judge whether image data is input from the receiving unit 11 through an interface (IF);
(2) If the input of the image data is satisfied in the decision step 1, step in the direction of step 2;
(3) If the input of the image data is not satisfied in the decision step 1, repeat the step 1;
(4) In the step 2, count the number of the black dots in the image data and at the same time transfer such data to the one block memory 4;
(5) In the step 3, judge whether the one block memory 4 is filled up with the image data;
(6) If the filling state of the one block memory is satisfied in the decision step 3, step in the direction of step 4;
(7) If the filling state of the one block memory is not satisfied in the decision step 3, repeat the step 2;
(8) In the step 4, judge whether a percentage of the black dots is higher than the predetermined level;
(9) If the percentage of the black dots is satisfied in the decision step 4, step in the direction of step 5;
(10) If the percentage of the black dots is not satisfied in the decision step 4, step in the direction of step 6.
(11) In the step 5, set a black flag (BF) to "1" and step in the direction of step 7;
(12) In the step 6, set the black flag (BF) to "zero" and step in the direction of the step 7.
(13) In the step 7, judge whether the recording control unit 7 is in a busy state.
(14) If it is in the busy state at the moment in the decision step 7, repeat the step 7;
(15) If it is not in the busy state in the decision step 7, step in the direction of step 8;
(16) In the step 8, output a print command for printing one block of the image data to the recording control unit 7 and also output a value of the BF as information to the recording control unit 7;
(17) In the step 9, judge whether the recording of the image data for one page is terminated:
(18) If it is terminated in the decision step 9, terminate the recording process; and
(19) If it is not terminated in the decision step 9, step in the direction of the step 1 and repeat the steps from 1 to 9.

In the above described process, one block of the image data is stored as dot image data which are decoded in the one block memory 4. For.example such block data is in a correspondence with a memory of 1728 × 48 (82944) dots when a semimulti-type recording head with 45 dots in one row and with a resolution of 8 dots/mm in a main scanning direction is used for forming an A-4 sized image.

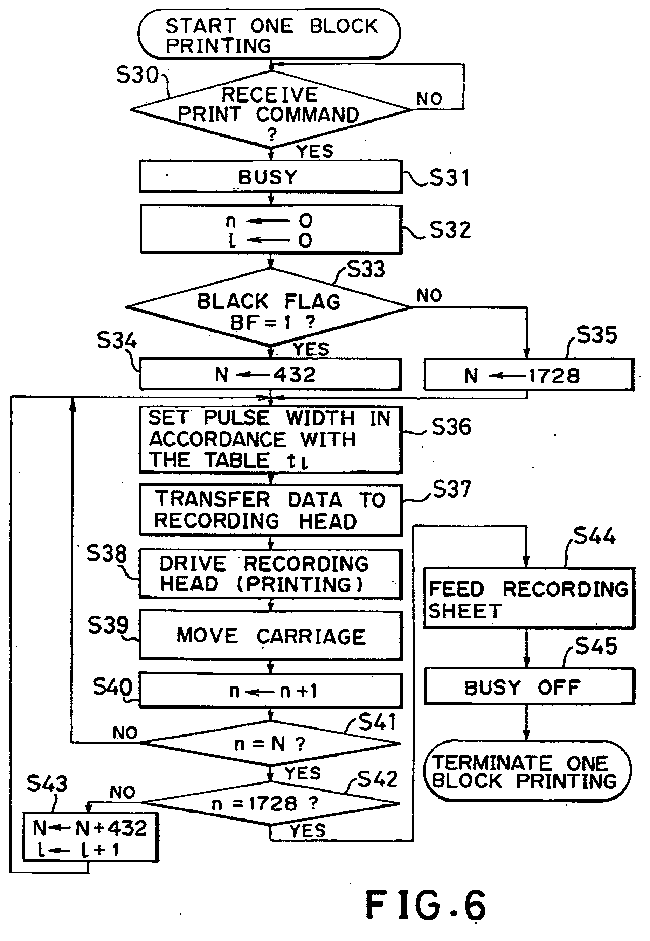

Fig.6 is a general flow chart of a recording process controlled by the recording control unit 7 for the one block recording. The recording process comprises the following steps:
(1) In step 30, judge whether the print command is received from the main control unit 1;
(2) If the print command is received in the decision step 30, step in the direction of step 31;
(3) If it is not received in the decision step 30, repeat the step 30;
(4) In the step 31, send a signal "BUSY" as a status signal to the main control unit 1 and step in the direction of step 32;
(5) In the step 32, define the values "n" and "l" of the counter as zero;
(6) In step 33, judge whether the black flag BF takes the value "1";
(7) If the black flag BF takes the value "1" (it means the high percentage of the black dots in one block of the image data) in the decision step 33, step in the direction of step 34;
(8) If the black flag does not take the value "1" in the decision step 33, step in the direction of step 35;
(9) In the step 34, insert "432" into "N" and step in the direction of step 36;
(10) In the step 35; insert "1728" into into "N" and step in the direction of step 36;
(11) In the step 36, determine an appropriate pulse width for driving the thermal elements in accordance with the relation between the pulse width and the temperature of the recording head (Fig. 4), but select t₀ (most wide) for the counter number l = 0 at first, and then step in the direction of step 37;
(12) In the step 37, transfer the image data for one row of the recording head 20 and step in the direction of step 38;
(13) In the step 38, drive the recording head 20 based on the one row of the image data and step in the direction of step 39;
(14) In the step 39, move the carriage along the scanning direction and print the one row of the image data, and then step in the direction of step 40;
(15) In the step 40, increment the value of the counter from "n" to "n + 1", and then step in the direction of step 41;
(16) In the step 41, judge whether the "n" is equal to "N";
(17) If the "n" is equal to "N" in the decision step 41, step in the direction of step 42;
(18) If the "n" is not equal to "N" in the decision step 41, step in the direction of step 36 and then repeat the steps 36-41;
(19) In the step 42, judge whether the "n" takes the value "1728";
(20) If the "n" takes the value "1728", step in the direction of step 44;
(21) If the "n" does not take the value "1728", step in the direction of step 43;
(22) In the step 43, increment the value of the counter from "N" to "n + 432" and "l" to "l + 1", and then step in the direction of the step 36 to repeat the steps 36-42;
(23) In the step 44, move the recording medium for one block and then step in the direction of step 45;
(24) In the step 45, turn the "BUSY" off and terminate the recording process for one block of the image data.

In the case of that the black flag BF takes "0" (i.e., BF=0, a low percentage of the black dots occupied in the image) in the step 33, repeat the steps 36-41 for 1728 times (corresponding to one line of the A4 sized image data) and then step in the direction of step 42.

In the case of that the black flag takes "1" (i.e., BF=1, percentage of the black dots occupied in the image is more than 50%), repeat the steps 36-41 for 432 times to record a first quarter of the block and then step in the direction of step 41. In this embodiment, the block is divided into four quarters which are independently recorded in turn with the pulse width table t₀,t₁, t₂ and t₃, respectively. Thus the pulse width is narrowed step by step in accordance with a progression of the recording. In addition, the ink ejection volume can be gradually reduced not only by limiting the pulse width but also by controlling a voltage or current to be supplied.

A stable recording image can be obtained by controlling the pulse width of the driving voltage used for printing the one block so as to make a pattern of the ink ejection uniform during the recording process.

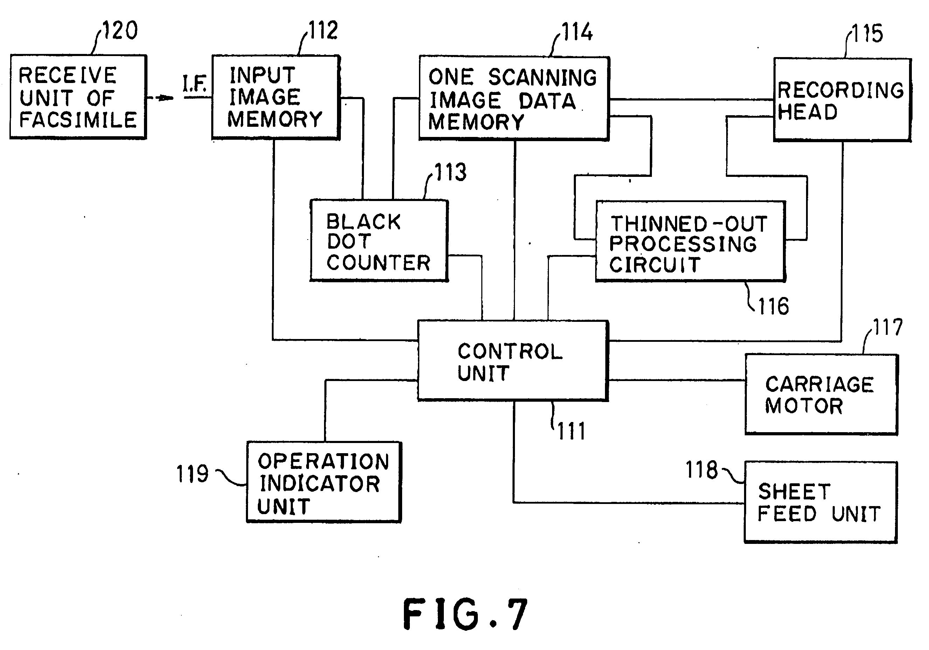

Fig. 7 is a block diagram showing an arrangement of a control portion in the recording apparatus of the second embodiment of the present invention.

Reference numeral 111 denotes a main control unit of the recording apparatus. The main control unit 111 is responsible for controlling the recording apparatus as a whole so as to perform data processing. The main control unit 111 includes ROM, RAM and CPU. In general, the CPU is connected to an ROM that stores various programs executed by the CPU, and to an RAM used as working areas (CPU, ROM and RAM are not shown in the figure). Reference numeral 112 denotes an input memory for accepting image data from a host apparatus functioning as the source of the image data. In this embodiment, the host apparatus is a receiving unit of the facsimile 120. The input memory 112 transfers the image data to a one-scanning image data memory 114 while a counter 113 counts the total number of black dots in one block of the image data. The black-dot counter 113 may be constituted by a hard circuit or performed by a software on the control unit 111. The memory for the one scanning movement of the recording head corresponds to a memory of non-coded image data (dot image data) of 48 × 1728 dots when the recording apparatus is provided so as to record A4 sized image with a resolution of 8 lines/mm. Reference numeral 116 denotes a thinned-out processing circuit which is responsible for removing some dots from the image data. The thinned-out process, for example make a checkered pattern, can be also performed by a software program on the control unit 111. In this case, it performs a logical multiplication (AND) every one line (48 dots) by using data of 01010101(55H) and 10101010(AAH) (0 means white data, 1 means black data) for every one bite of the dot image data. Using data of 00000000 (00H) for the logical multiplication every one line (48 dots), spaces are formed every one line.

There are two paths from the memory 114 to the recording head, i.e., one by which the image data is directly transferred and the other one by which the image data is transferred through the thinned-out processing circuit. These paths can be selected by the control unit 111.

Reference numeral 117 denotes a carriage motor of the recording head (corresponding to reference numeral 17 in Fig. 2). The carriage motor is responsible for moving the carriage in the main-scanning direction with transferring the data every one row to the recording head when the image data is prepared enough to perform the one scanning movement of the recording head. In this embodiment, the carriage moves in one direction simultaneously with that the recording head records the image data for the one scanning. However, it is also possible to record such data by moving the carriage back and forth.

Reference numeral 118 denotes a unit for transporting the recording medium in the direction perpendicular to moving direction of the recording head. The unit 118 is composed of a stepping motor and a motor-driver circuit which are responsible for transporting the recording medium in the sub-scanning direction step by step for each scanning movement. The transporting of the recording medium can be performed every end of the scanning movements.

Reference numeral 119 denotes a unit for indicating operating conditions of the recording apparatus. The unit 119 is composed of operation switches, means for indicating the operating conditions such as an error indicator, means for indicating the condition of the power supply such as a switch-on indicator, and the like.

Reference numeral 120 denotes a receiving unit of the facsimile which is responsible for receiving the data through a line and transporting the input data to the input image memory 112.

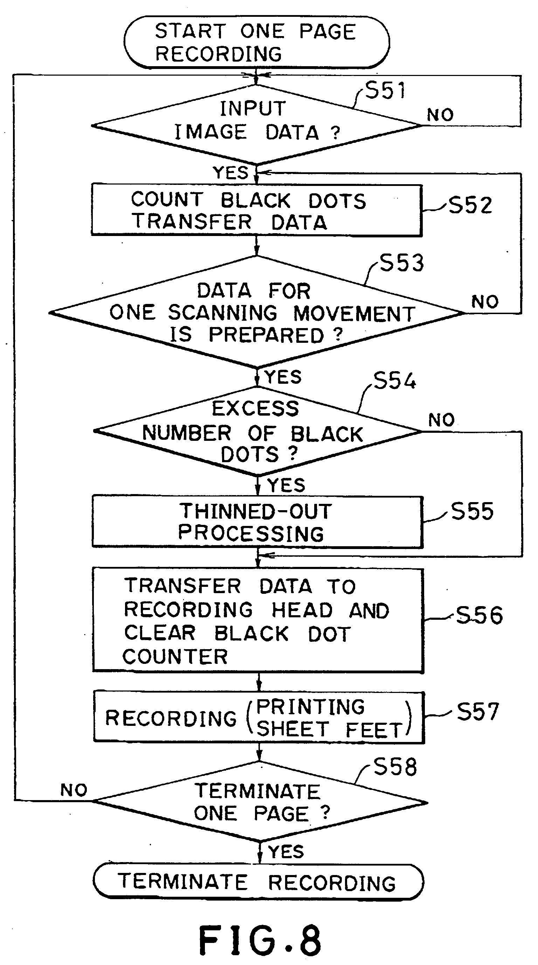

Fig. 8 is a general flow chart of a recording process controlled by the control unit 111 of the second embodiment. The recording procedure comprises the following steps:
(1) In step 51, judge whether the image data is transferred from the receiving unit 120 to the input image data memory 112 via an interface IF;
(2) In the input of the image data is satisfied in the decision step 51, step in the direction of step 52;
(3) If the input of the image data is not satisfied in the decision step 51, repeat the same step;
(4) In the step 52, count the number of the black dots in the input data and at the same time transfer the image data to one scanning image data memory 114;
(5) In the step 53, judge whether a quantity of the input data is enough to perform one scanning movement of the recording head, i.e., judge whether the one scanning image data memory 114 is filled up with the image data:
(6) If the quantity of the input data is satisfied in the decision step 53, step in direction of step 54;
(7) If the quantity of the input data is not satisfied in decision step 53, repeat the step 53;
(8) In the step 54, judge whether a percentage of the black dots is higher than the predetermined level;
(9) If the percentage of the black dots is satisfied in the decision step 54, step in the direction of step 55;
(10) If the percentage of the black dots is not satisfied in the decision step 54, step in the direction of step 56;
(11) In the step 55, thin out dots in the image data and step in the direction of step 56;
(12) In the step 56, transfer the image data to the recording head 20 and reset the black dot counter 113, and then step in the direction of step 57;
(13) In the step 57, print the image in accordance with the image data and step in the direction of step 58;
(14) In the step 58, judge whether the printing of the image data for one page is terminated;
(15) If the printing is terminated in the decision step 58, terminate the recording procedure; and
(16) If it is not terminated in decision step 9, step in the direction of the step 51 and repeat the steps from 51 to 58.

In the above described process, the memory 112 stores the image data in the form of letter codes or coded data such as MH, MR, MMR and the like, while the memory 114 stores the image data in the form of non-coded data (dot image data). In the case of the shuttle-type recording apparatus, the printing can be started after filling up the memory 114 with the input data so as to move the carriage smoothly. For example, a total quantity of dots to be printed by the recording head with 48 dots for printing an image (8 pel. A-4 size) is corresponded with one scanning data of 48 × 1728 = 82944. Therefore, in the case of performing the thinning-out process at over 70% of black dots, step in the direction of the step 55 when the number of the black dots is 58061 or over, while step in the direction of step 56 when the number of the black dots is under 58061. In this embodiment, patterns of the thinned-out processing, a standard percentage of the black dots or the like can be defined arbitrarily.

### VARIOUS ASPECTS OF THE INVENTION

The present invention can be applied to a facsimile using an ink-jet recording apparatus of piezo-type as its recording system in which piezoelectric elements are used as elements for generating ink-ejection energy. The present invention is particularly suitably usable in an ink-jet recording head having heating elements that produce thermal energy as energy used for ink ejection and recording apparatus using the head. This is because, the high density of the picture element, and the high resolution of the recording are possible.

The typical structure and the principle are preferably the one disclosed in U.S. Patent Nos. 4,723,129 and 4,740,796. The principle is applicable to a so-called on-demand type recording system and a continuous type recording system particularly however, it is suitable for the on-demand type because the principle is such that at least one driving signal is applied to an electrothermal transducer disposed on liquid (ink) retaining sheet or liquid passage, the driving signal being enough to provide such a quick temperature rise beyond a departure from nucleation boiling point, by which the thermal energy is provide by the electrothermal transducer to produce film boiling on the heating portion of the recording head, .whereby a bubble can be formed in the liquid (ink) corresponding to each of the driving signals. by development and collapse of the bubble, the liquid (ink) is ejected through an ejection outlet to produce at least one droplet. The driving signal is preferably in the form of a pulse, because the development and collapse of the bubble can be effected instantaneously, and therefore, the liquid (ink) is ejected with quick response. The driving signal in the form of the pulse is preferably such as disclosed in U.S. Patent Nos. 4,463,359 and 4,345,262. In addition, the temperature increasing rate of the heating surface is preferably such as disclosed in U.S. Patent No. 4,313,124.

The structure of the recording head may be as shown in U.S. Patents Nos. 4,558,33 and 4,459,600 wherein the heating portion is disposed at a bent portion in addition to the structure of the combination of the ejection outlet, liquid passage and the electrothermal transducer as disclosed in the above-mentioned patents. In addition, the present invention is applicable to the structure disclosed in Japanese Patent Application Laying-Open No. 123670/1984 wherein a common slit is used as the ejection outlet for a plurality of electrothermal transducers, and to the structure disclosed in Japanese Patent Application Laying-Open No. 138461/1984 wherein an opening for absorbing pressure wave of the thermal energy is formed corresponding to the ejecting portion. This is because, the present invention is effective to perform the recording operation with certainty and at high efficiency irrespective of the type of the recording head.

The present invention is effectively applicable to a so-called full-line type recording head having a length corresponding to the maximum recording width. Such a recording head may comprise a single recording head and a plurality recording head combined to cover the entire width.

In addition, the present invention is applicable to a serial type recording head wherein the recording head is fixed on the main assembly, to a replaceable chip type recording head which is connected electrically with the main apparatus and can be supplied with the ink by being mounted in the main assembly, or to a cartridge type recording head having an integral ink container.

The provision of recovery means and the auxiliary means for the preliminary operation are preferable, because they can further stabilize the effect of the present invention. As for such means, there are capping means for the recording head, cleaning means therefor, pressing or sucking means, preliminary heating means by the ejection electrothermal transducer or by a combination of the ejection electrothermal transducer and additional heating element and means for preliminary ejection not for the recording operation, which can stabilize the recording operation.

As a regards the kinds and the number of the recording heads mounted, a single head corresponding to a single color ink may be equipped, or plurality of heads corresponding respectively to a plurality of ink materials having different recording color or density may be equipped.

The present invention has been described in detail with respect to preferred embodiments, and it will now be that changes and modifications may be made without departing from the invention in its broader aspects, and it is the invention, therefore, in the appended claims to cover all such changes and modifications.

## Claims

1. An ink jet recording apparatus for recording an image on a recording medium in accordance with image data which includes data representing dots to be printed and data representing where dots are not to be printed on the recording medium by using a recording head (20) having a plurality of aligned recording elements, comprising:
control means (1,7) for selecting one of at least a first recording mode and a second recording mode in which the recording head ejects less ink to record the predetermined amount of image data than if the first recording mode were used, characterised by computing means (3) operable to determine from the predetermined amount of image data the number of dots to be printed by the recording head in accordance with the predetermined amount of image data before printing occurs, and the control means (1,7) being arranged to cause the predetermined amount of image data to be recorded using the second recording mode when the computing means determines that the number of dots to be printed in accordance with the predetermined amount of image data exceeds a predetermined number.

2. An ink jet recording apparatus according to claim 1 in which the control means is operable when the first recording mode is selected to supply constant energy to the recording element during recording of the predetermined amount of image data and when the second recording mode is selected, the control means is operable to reduce the energy applied to the recording elements in the course of recording the predetermined amount of image data.

3. An ink-jet recording apparatus as in any preceding claim, wherein the selecting means is operable to select one of the first mode and the second mode each time the predetermined amount of image data is recorded.

4. An ink-jet recording apparatus as in claims 1, 2, or 3, further comprises moving means (16) for relatively moving the recording head (20) with respect to the recording medium in order to record the predetermined amount of image data, and wherein, in the second recording mode, the control means (1,7) is operable to gradually reduce energy to be supplied to the recording elements in order to control driving of the recording head (20) during a relative movement of the recording head (20) caused by the moving means (16).

5. An ink-jet recording apparatus as in claim 4, wherein a direction of the relative movement effected by the moving means differs from the direction in which the plural recording elements of the recording head (20) are arranged.

6. An ink-jet recording apparatus according to any preceding claim, wherein, when the computed value obtained by the computing means (3) exceeds the predetermined number, the control means (1,7) selects a recording mode in which an image is recorded by thinning predetermined printing dots associated with the predetermined amount of image data, and when the computed value obtained by the computing means (3) does not exceed the predetermined number, the control means (1,7) selects a recording mode in which an image is recorded without thinning the predetermined printing dots.

7. An ink-jet recording apparatus as in any of claims 4 to 6, wherein the predetermined amount of image data is equal to an amount of image data which can be recorded in one recording scan effected by the recording head (20).

8. An ink-jet recording apparatus according to any preceding claim, wherein the recording head (20) is arranged to eject ink droplets by using thermal energy to cause a change in state of the ink.

9. An ink jet recording apparatus according to any preceding claim in which the predetermined number of dots to be printed in accordance with the predetermined amount of image data is 50% of the total dots for the predetermined amount of image data.

10. An ink jet recording apparatus according to any preceding claim in which the selecting means is operable to select one of the first mode and the second mode at predetermined intervals during the recording of the predetermined amount of image data.

11. An ink jet recording method for recording an image on a recording medium in accordance with image data which includes data representing dots to be printed and data representing where dots are not to be printed on the recording medium by using a recording head (20) having a plurality of aligned recording elements, comprising the steps of:
selecting (S4, S54) one of at least a first recording mode and a second recording mode in which the recording head ejects less ink to record the predetermined amount of image data than if the first recording mode were used, characterised by the step (S2, S52) of determining from the predetermined amount of image data the number of dots to be printed by the recording head in accordance with the predetermined amount of image data before printing occurs, and causing (S8, S56) the predetermined amount of image data to be recorded using the second recording mode when the number of dots to be printed in accordance with the predetermined amount of image data exceeds a predetermined number.

12. A ink jet recording method as in claim 11, wherein in the first recording mode, constant energy is supplied to the recording elements during recording of the predetermined amount of image data, and in the second recording mode, energy which is supplied to the recording elements is reduced in the course of recording the predetermined amount of image data.

13. An ink jet recording method as in claim 11 or claim 12, wherein a recording mode is selected each time the predetermined amount of image data is recorded.

14. An ink jet recording method according to any of claims 11 to 13, in which the recording head (20) is moved with respect to the recording medium in order to record the predetermined amount of image data, and wherein, in the second recording mode, the energy supplied to the recording elements is gradually reduced in order to control driving of the recording head (20) during a given relative movement of the recording head (20).

15. An ink jet recording method as in claim 14, wherein a direction of the relative movement differs from a direction in which the plural recording elements of the recording head (20) are arranged.

16. An ink jet recording method according to any of claims 10 to 15 in which when the number of dots to be printed by the recording head in accordance with the predetermined amount of data exceeds the predetermined number, a recording mode is selected in which an image is recorded by thinning predetermined printing dots associated with the predetermined amount of image data, and when the number of dots to be printed by the recording head in accordance with the predetermined amount of data does not exceed the predetermined number, a recording mode in which an image is recorded without thinning a predetermined printing dots is selected.

17. An ink jet recording method according to any of claims 14 to 16, wherein the predetermined amount of image data is equal to an amount of data which can be recorded by one recording scan resulting from the relative movement of the recording head (20).

18. An ink jet recording method as in any of claims 11 to 17, wherein the recording head (20) ejects ink by using thermal energy to cause a change in state of the ink.

19. An ink jet recording method according to any of claims 11 to 18, in which the predetermined number of dots to be printed in accordance with the predetermined amount of image data is 50% of the total dots for the predetermined amount of image data.

20. An ink jet recording method according to any preceding claims in which the step of selecting one of the first mode or the second mode is carried out at predetermined intervals during the recording of the predetermined amount of image data.

## Patentansprüche

1. Tintenstrahlaufzeichnungsvorrichtung zur Aufzeichnung eines Bilds auf einem Aufzeichnungsmedium in Übereinstimmung mit Bilddaten, welche zu druckende Punkte repräsentierende Daten, und Daten, die repräsentieren, wo keine Punkte auf das Aufzeichnungsmedium zu drucken sind, enthalten, unter Anwendung eines Aufzeichnungskopfs (20), der eine Vielzahl von ausgerichteten Aufzeichnungselementen hat, mit
einer Steuereinrichtung (1, 7) zur Auswahl eines von mindestens einem ersten Aufzeichnungsmodus und einem zweiten Aufzeichnungsmodus, bei welchem der Aufzeichnungskopf weniger Tinte, als wenn der erste Aufzeichnungsmodus benutzt würde, ausstößt, um die festgelegte Menge von Bilddaten aufzuzeichnen, gekennzeichnet durch eine Recheneinrichtung (3), die betätigbar ist, um aus der festgelegten Menge an Bilddaten die Anzahl an Punkten, die mittels des Aufzeichnungskopfs in Übereinstimmung mit der festgelegten Menge an Bilddaten zu drucken ist, zu bestimmen, bevor der Druck eintritt, und wobei die Steuereinrichtung (1, 7) derart eingerichtet ist, daß die Aufzeichnung der festgelegten Menge an Bilddaten unter Nutzung des zweiten Aufzeichnungsmodus bewirkt wird, wenn die Recheneinrichtung feststellt, daß die Anzahl an in Übereinstimmung mit der festgelegten Menge an Bilddaten zu druckenden Punkten eine festgelegte Anzahl überschreitet.

2. Tintenstrahlaufzeichnungsvorrichtung gemäß Anspruch 1, bei welcher die Steuereinrichtung, wenn der erste Aufzeichnungsmodus gewählt ist, betätigbar ist, um dem Aufzeichnungselement während der Aufzeichnung der festgelegten Menge an Bilddaten eine konstante Energie zuzuführen, und die Steuereinrichtung, wenn der zweite Aufzeichnungsmodus gewählt ist, betätigbar ist, um die den Aufzeichnungselementen zugeführte Energie im Verlauf der Aufzeichnung der festgelegten Menge an Bilddaten zu reduzieren.

3. Tintenstrahlaufzeichnungsvorrichtung gemäß einem vorhergehenden Anspruch, bei welcher die Auswahleinrichtung betätigbar ist, um den ersten Modus oder den zweiten Modus jedesmal auszuwählen, wenn die festgelegte Menge an Bilddaten aufgezeichnet wird.

4. Tintenstrahlaufzeichnungsvorrichtung gemäß Anspruch 1, 2 oder 3, die ferner eine Bewegungseinrichtung (16) zur Relativbewegung des Aufzeichnungskopfs (20) mit Bezug auf das Aufzeichnungsmedium aufweist, um die festgelegte Menge an Bilddaten aufzuzeichnen, und bei welcher die Steuereinrichtung (1, 7) in dem zweiten Aufzeichnungsmodus betätigbar ist, um die den Aufzeichnungselementen zuzuführende Energie allmählich zu reduzieren, um den Antrieb des Aufzeichnungskopfs (20) während einer durch die Bewegungseinrichtung (16) bewirkten Relativbewegung des Aufzeichnungskopfs (20) zu steuern.

5. Tintenstrahlaufzeichnungsvorrichtung gemäß Anspruch 4, bei welcher sich eine Richtung der durch die Bewegungseinrichtung bewirkten Relativbewegung von der Richtung unterscheidet, in welcher die mehreren Aufzeichnungselemente des Aufzeichnungskopfs (20) angeordnet sind.

6. Tintenstrahlaufzeichnungsvorrichtung gemäß einem vorhergehenden Anspruch, bei welcher, wenn der mittels der Recheneinrichtung (3) erzielte berechnete Wert die festgelegte Anzahl überschreitet, die Steuereinrichtung (1, 7) einen Aufzeichnungsmodus wählt, in welchem ein Bild durch Ausdünnen festgelegter Druckpunkte aufgezeichnet wird, die mit der festgelegten Menge an Bilddaten verbunden sind, und, wenn der mittels der Recheneinrichtung (3) erzielte berechnete Wert die festgelegte Anzahl nicht überschreitet, die Steuereinrichtung (1, 7) einen Aufzeichnungsmodus wählt, in welchem ein Bild ohne Ausdünnen der festgelegten Druckpunkte aufgezeichnet wird.

7. Tintenstrahlaufzeichnungsvorrichtung gemäß einem der vorhergehenden Ansprüche 4 bis 6, bei welcher die festgelegte Menge an Bilddaten gleich einer Menge an Bilddaten ist, welche bei einer mittels des Aufzeichnungskopfs (20) bewirkten Aufzeichnungsabtastung aufgezeichnet werden kann.

8. Tintenstrahlaufzeichnungsvorrichtung gemäß einem vorhergehenden Anspruch, bei welcher der Aufzeichnungskopf (20) eingerichtet ist, um Tintentröpfchen unter Anwendung thermischer Energie, um eine Zustandsänderung der Tinte zu verursachen, auszustoßen.

9. Tintenstrahlaufzeichnungsvorrichtung gemäß einem vorhergehenden Anspruch, bei welcher die festgelegte Anzahl an in Übereinstimmung mit der festgelegten Menge an Bilddaten zu druckenden Punkten 50% der gesamten Punkte für die festgelegte Menge an Bilddaten beträgt.

10. Tintenstrahlaufzeichnungsvorrichtung gemäß einem vorhergehenden Anspruch, bei welcher die Auswahleinrichtung betätigbar ist, um den ersten Modus oder den zweiten Modus während der Aufzeichnung der festgelegten Menge an Bilddaten in festgelegten Intervallen auszuwählen.

11. Tintenstrahlaufzeichnungsverfahren zur Aufzeichnung eines Bilds auf einem Aufzeichnungsmedium in Übereinstimmung mit Bilddaten, welche zu druckende Punkte repräsentierende Daten, und Daten, die repräsentieren, wo keine Punkte auf das Aufzeichnungsmedium zu drucken sind, enthalten, unter Anwendung eines Aufzeichnungskopfs (20), der eine Vielzahl von ausgerichteten Aufzeichnungselementen hat, das die Schritte aufweist,
Auswahl (S4, S54) eines von mindestens einem ersten Aufzeichnungsmodus und einem zweiten Aufzeichnungsmodus, bei welchem der Aufzeichnungskopf weniger Tinte, als wenn der erste Aufzeichnungsmodus benutzt würde, ausstößt, um die festgelegte Menge an Bilddaten aufzuzeichnen, gekennzeichnet durch den Schritt (S2, S52) der Bestimmung der Anzahl an Punkten, die mittels des Aufzeichnungskopfs in Übereinstimmung mit der festgelegten Menge an Bilddaten zu drucken ist, bevor der Druck eintritt, aus der festgelegten Menge an Bilddaten, und Bewirken (S8, S56), daß die festgelegte Menge an Bilddaten unter Nutzung des zweiten Aufzeichnungsmodus aufgezeichnet wird, wenn die Anzahl an in Übereinstimmung mit der festgelegten Menge an Bilddaten zu druckenden Punkten eine festgelegte Anzahl überschreitet.

12. Tintenstrahlaufzeichnungsverfahren gemäß Anspruch 11, bei welchem in dem ersten Aufzeichnungsmodus den Aufzeichnungselementen während der Aufzeichnung der festgelegten Menge an Bilddaten eine konstante Energie zugeführt wird, und in dem zweiten Aufzeichnungsmodus die Energie, welche den Aufzeichnungselementen zugeführt wird, im Verlauf der Aufzeichnung der festgelegten Menge an Bilddaten reduziert wird.

13. Tintenstrahlaufzeichnungsverfahren gemäß Anspruch 11 oder Anspruch 12, bei welchem ein Aufzeichnungsmodus jedesmal ausgewählt wird, wenn die festgelegte Menge an Bilddaten aufgezeichnet wird.

14. Tintenstrahlaufzeichnungsverfahren gemäß einem der Ansprüche 11 bis 13, bei welchem der Aufzeichnungskopf (20) mit Bezug auf das Aufzeichnungsmedium bewegt wird, um die festgelegte Menge an Bilddaten aufzuzeichnen, und bei welchem in dem zweiten Aufzeichnungsmodus die den Aufzeichnungselementen zugeführte Energie allmählich reduziert wird, um den Antrieb des Aufzeichnungskopfs (20) während einer gegebenen Relativbewegung des Aufzeichnungskopfs (20) zu steuern.

15. Tintenstrahlaufzeichnungsverfahren gemäß Anspruch 14, bei welchem sich eine Richtung der Relativbewegung von einer Richtung unterscheidet, in welcher die mehreren Aufzeichnungselemente des Aufzeichnungskopfs (20) angeordnet sind.

16. Tintenstrahlaufzeichnungsverfahren gemäß einem der Ansprüche 10 bis 15, bei welchem, wenn die mittels des Aufzeichnungskopfs in Übereinstimmung mit der festgelegten Menge an Daten zu druckende Anzahl an Punkten die festgelegte Anzahl überschreitet, ein Aufzeichnungsmodus gewählt wird, in welchem ein Bild durch Ausdünnen festgelegter Druckpunkte aufgezeichnet wird, die mit der festgelegten Menge an Bilddaten verbunden sind, und, wenn die mittels des Aufzeichnungskopfs in Übereinstimmung mit der festgelegten Menge an Daten zu druckende Anzahl an Punkten die festgelegte Anzahl nicht überschreitet, ein Aufzeichnungsmodus gewählt wird, in welchem ein Bild ohne Ausdünnen von festgelegten Druckpunkten aufgezeichnet wird.

17. Tintenstrahlaufzeichnungsverfahren gemäß einem der Ansprüche 14 bis 16, bei welchem die festgelegte Menge an Bilddaten gleich einer Menge an Bilddaten ist, welche mittels einer aus der Relativbewegung des Aufzeichnungskopfs (20) resultierenden Aufzeichnungsabtastung aufgezeichnet werden kann.

18. Tintenstrahlaufzeichnungsverfahren gemäß einem der Ansprüche 11 bis 17, bei welchem der Aufzeichnungskopf (20) Tinte unter Anwendung thermischer Energie, um eine Zustandsänderung der Tinte zu verursachen, ausstößt.

19. Tintenstrahlaufzeichnungsverfahren gemäß einem der Ansprüche 11 bis 18, bei welchem die festgelegte Anzahl an in Übereinstimmung mit der festgelegten Menge an Bilddaten zu druckenden Punkten 50% der gesamten Punkte für die festgelegte Menge an Bilddaten beträgt.

20. Tintenstrahlaufzeichnungsverfahren gemäß einem der vorhergehenden Ansprüche, bei welchem der Schritt der Auswahl des ersten Modus oder des zweiten Modus während der Aufzeichnung der festgelegten Menge an Bilddaten in festgelegten Intervallen ausgeführt wird.

## Revendications

1. Appareil d'enregistrement par jets d'encre destiné à enregistrer une image sur un support d'enregistrement conformément à des données d'image qui comprennent des données représentant des points devant être imprimés et des données représentant où des points ne doivent pas être imprimés sur le support d'enregistrement, en utilisant une tête d'enregistrement (20) ayant plusieurs éléments alignés d'enregistrement, comportant :
des moyens de commande (1, 7) destinés à sélectionner l'un d'au moins un premier mode d'enregistrement et un second mode d'enregistrement dans lequel la tête d'enregistrement éjecte moins d'encre pour enregistrer la quantité prédéterminée de données d'image que si le premier mode d'enregistrement était utilisé, caractérisé par des moyens de calcul (3) pouvant être mis en oeuvre pour déterminer, à partir de la quantité prédéterminée de données d'image, le nombre de points devant être imprimés par la tête d'enregistrement conformément à la quantité prédéterminée de données d'image avant qu'une impression ait lieu, et les moyens de commande (1, 7) étant agencés pour provoquer l'enregistrement de la quantité prédéterminée de données d'image en utilisant le second mode d'enregistrement lorsque les moyens de calcul déterminent que le nombre de points devant être imprimés conformément à la quantité prédéterminée de données d'image dépasse un nombre prédéterminé.

2. Appareil d'enregistrement par jets d'encre selon la revendication 1, dans lequel les moyens de commande peuvent être mis en oeuvre lorsque le premier mode d'enregistrement est sélectionné pour fournir une énergie constante à l'élément d'enregistrement pendant l'enregistrement de la quantité prédéterminée de données d'image, et lorsque le second mode d'enregistrement est sélectionné, les moyens de commande peuvent être mis en oeuvre pour réduire l'énergie appliquée aux éléments d'enregistrement au cours de l'enregistrement de la quantité prédéterminée de données d'image.

3. Appareil d'enregistrement par jets d'encre selon l'une quelconque des revendications précédentes, dans lequel les moyens de sélection peuvent être mis en oeuvre pour sélectionner l'un du premier mode et du second mode à chaque fois que la quantité prédéterminée de données d'image est enregistrée.

4. Appareil d'enregistrement par jets d'encre selon les revendications 1, 2 ou 3, qui comporte en outre des moyens (16) de déplacement destinés à déplacer relativement la tête d'enregistrement (20) par rapport au support d'enregistrement pour enregistrer la quantité prédéterminée de données d'image, et dans lequel, dans le second mode d'enregistrement, les moyens de commande (1, 7) peuvent être mis en oeuvre pour réduire progressivement l'énergie devant être fournie aux éléments d'enregistrement afin de commander l'attaque de la tête d'enregistrement (20) pendant un mouvement relatif de la tête d'enregistrement (20) provoqué par les moyens de déplacement (16).

5. Appareil d'enregistrement par jets d'encre selon la revendication 4, dans lequel la direction du mouvement relatif effectué par les moyens de déplacement diffère de la direction dans laquelle les éléments d'enregistrement de la tête d'enregistrement (20) sont disposés.

6. Appareil d'enregistrement par jets d'encre selon l'une quelconque des revendications précédentes, dans lequel, lorsque la valeur calculée obtenue par les moyens de calcul (3) dépasse le nombre prédéterminé, les moyens de commande (1, 7) sélectionnent un mode d'enregistrement dans lequel une image est enregistrée par éclaircissage portant sur des points d'impression prédéterminés associés à la quantité prédéterminée de données d'image, et lorsque la valeur calculée obtenue par les moyens de calcul (3) ne dépassse pas le nombre prédéterminé, les moyens de commande (1, 7) sélectionnent un mode d'enregistrement dans lequel une image est enregistrée sans éclaircissage des points d'impression prédéterminés.

7. Appareil d'enregistrement par jets d'encre selon l'une quelconque des revendications 4 à 6, dans lequel la quantité prédéterminée de données d'image est égale à une quantité de données d'image qui peut être corrigée dans un balayage d'enregistrement effectué par la tête d'enregistrement (20).

8. Appareil d'enregistrement par jets d'encre selon l'une quelconque des revendications précédentes, dans lequel la tête d'enregistrement (20) est agencée de façon à éjecter des gouttelettes d'encre en utilisant de l'énergie thermique pour provoquer un changement d'état de l'encre.

9. Appareil d'enregistrement par jets d'encre selon l'une quelconque des revendications précédentes, dans lequel le nombre prédéterminé de points devant être imprimés conformément à la quantité prédéterminée de données d'image est égal à 50 % du total des points pour la quantité prédéterminée de données d'image.

10. Appareil d'enregistrement par jets d'encre selon l'une quelconque des revendications précédentes, dans lequel les moyens de sélection peuvent être mis en oeuvre pour sélectionner l'un du premier mode et du second mode à intervalles prédéterminés pendant l'enregistrement de la quantité prédéterminée de données d'image.

11. Procédé d'enregistrement par jets d'encre pour enregistrer une image sur un support d'enregistrement conformément à des données d'image qui comprennent des données représentant des points devant être imprimés et des données représentant des positions où des points ne doivent pas être imprimés sur le support d'enregistrement, en utilisant une tête d'enregistrement (20) ayant plusieurs éléments alignés d'enregistrement, comprenant les étapes dans lesquelles :
on sélectionne (S4, S54) l'un d'au moins un premier mode d'enregistrement et un second mode d'enregistrement dans lequel la tête d'enregistrement éjecte moins d'encre pour enregistrer la quantité prédéterminée de données d'image que si le premier mode d'enregistrement était utilisé, caractérisé par l'étape (S2, S52) de détermination, à partir de la quantité prédéterminée de données d'image, du nombre de points devant être imprimés par la tête d'enregistrement conformément à la quantité prédéterminée de données d'image avant qu'une impression ait lieu, et l'enregistrement (S8, S56) de la quantité prédéterminée de données d'image en utilisant le second mode d'enregistrement lorsque le nombre de points devant être imprimés conformément à la quantité prédéterminée de données d'image dépasse un nombre prédéterminé.

12. Procédé d'enregistrement par jets d'encre selon la revendication 11, dans lequel, dans le premier mode d'enregistrement, de l'énergie constante est fournie aux éléments d'enregistrement pendant l'enregistrement de la quantité prédéterminée de données d'image, et, dans le second mode d'enregistrement, l'énergie qui est fournie aux éléments d'enregistrement est réduite au cours de l'enregistrement de la quantité prédéterminée de données d'image.

13. Procédé d'enregistrement par jets d'encre selon la revendication 11 ou la revendication 12, dans lequel un mode d'enregistrement est sélectionné à chaque fois que la quantité prédéterminée de données d'image est enregistrée.

14. Procédé d'enregistrement par jets d'encre selon l'une quelconque des revendications 11 à 13, dans lequel la tête d'enregistrement (20) est déplacée par rapport au support d'enregistrement pour enregistrer la quantité prédéterminée de données d'image, et dans lequel, dans le second mode d'enregistrement, l'énergie fournie aux éléments d'enregistrement est diminuée progressivement afin de commander l'attaque de la tête d'enregistrement (20) pendant un mouvement relatif donné de la tête d'enregistrement (20).

15. Procédé d'enregistrement par jets d'encre selon la revendication 14, dans lequel la direction du mouvement relatif diffère de la direction dans laquelle les éléments d'enregistrement de la tête d'enregistrement (20) sont disposés.

16. Procédé d'enregistrement par jets d'encre selon l'une quelconque des revendications 10 à 15, dans lequel, lorsque le nombre de points devant être imprimés par la tête d'enregistrement conformément à la quantité prédéterminée de données dépasse le nombre prédéterminé, on sélectionne un mode d'enregistrement dans lequel l'image est enregistrée par un éclaircissage portant sur des points d'impression prédéterminés associés à la quantité prédéterminée de données d'image, et lorsque le nombre de points devant être imprimés par la tête d'enregistrement conformément à la quantité prédéterminée de données ne dépasse pas le nombre prédéterminé, on sélectionne un mode d'enregistrement dans lequel une image est enregistrée sans éclaircissage de points d'impression prédéterminés.

17. Procédé d'enregistrement par jets d'encre selon l'une quelconque des revendications 14 à 16, dans lequel la quantité prédéterminée de données d'image est égale à une quantité de données qui peut être enregistrée par un balayage d'enregistrement résultant du mouvement relatif de la tête d'enregistrement (20).

18. Procédé d'enregistrement par jets d'encre selon l'une quelconque des revendications 11 à 17, dans lequel la tête d'enregistrement (20) éjecte de l'encre en utilisant de l'énergie thermique pour provoquer un changement d'état de l'encre.

19. Procédé d'enregistrement par jets d'encre selon l'une quelconque des revendications 11 à 18, dans lequel le nombre prédéterminé de points devant être imprimés conformément à la quantité prédéterminée de données d'image est égal à 50 % du total des points pour la quantité prédéterminée de données d'image.

20. Procédé d'enregistrement par jets d'encre selon l'une quelconque des revendications précédentes, dans lequel l'étape de sélection de l'un du premier mode ou du second mode est exécutée à intervalles prédéterminés pendant l'enregistrement de la quantité prédéterminée de données d'image.
